# EUROPEAN PATENT SPECIFICATION

(11) **EP 4 059 621 B1**
(45) Date of publication and mention of the grant of the patent: **15.10.2025**
(21) Application number: 22161980.2
(22) Date of filing: 14.03.2022
(51) Int. Cl.: B06B 1/06, A61B 8/00

(54) **ULTRASONIC PROBE**
ULTRASCHALLSONDE
SONDE À ULTRASONS

(30) Priority: 16.03.2021 JP 2021042225
(43) Date of publication of application: 21.09.2022
(73) Proprietor: FUJIFILM Corporation, Tokyo 106-8620 (JP)
(72) Inventor: KOBAYASHI, Kazuhiro, Chiba, 277-0804 (JP); SAWADA, Wataru, Chiba, 277-0804 (JP); WATANABE, Toru, Chiba, 277-0804 (JP)
(74) Representative: Strehl & Partner mbB

(56) References cited:
- EP-A1- 3 787 314
- EP-A2- 2 842 642
- US-A1- 2012 238 880
- US-A1- 2019 307 424

## Description

### TECHNICAL FIELD

The present disclosure relates to an ultrasonic probe and to a method of manufacturing a backing, and in particular, to a structure and a manufacturing method of the backing.

### BACKGROUND

In ultrasonic diagnoses, ultrasonic probes are used. An ultrasonic probe includes an ultrasound transmission/reception face. The ultrasonic probe transmits ultrasound into a living body and receives ultrasound reflected from within the body while the ultrasound transmission/reception face of the ultrasonic probe is held in contact with the surface of the subject body.

The ultrasonic probe also includes a transducer assembly configured with, for example, a backing (a soft backing), a piezoelectric layer (a piezoelectric element array, a transducer layer), a matching layer (a matching element array), and an acoustic lens disposed in this order from the back (farthest from the living body) to the front (nearest to the body). A hard backing layer (a hard backing element array) may be disposed between the backing and the piezoelectric layer.

The backing attenuates ultrasound that propagates backward from the back of the transducer layer (or the back of the hard backing). Conventionally, the composition of the backing has been optimized to absorb ultrasound within the backing such that ultrasound is sufficiently attenuated. Although two types of ultrasound attenuation, absorption attenuation and scattering attenuation, are available, in most cases the conventional backing uses absorption attenuation to attenuate the backward ultrasound.

JP 2015-221214 A discloses an ultrasonic probe which includes a semiconductor substrate with a capacitive micro-machined ultrasound transducer (CMUT), a stack of a first backing layer, and a second backing layer. JP 2015-221214 A describes in paragraph [0035] that the first backing layer is made from porous ceramic which is filled with a resin. (US 2013/0285174 A1 discloses a similar structure in paragraph [0047].) JP 2015-221214 A further describes in paragraph [0035] that a reason to use the porous ceramic is to match the linear expansion coefficient of the first backing layer to that of the semiconductor substrate. However, JP 2015-221214 A (also US 2013/0285174 A1) nowhere discloses a specific structure or effects of the porous ceramic, or a transducer layer made from a piezoelectric material.

An ultrasound probe having the features in the pre-characterizing portion of claim 1 is disclosed in US 2019/307424 A1. Further ultrasound probes related to the one of the present invention are disclosed in EP 2 842 642 A2, EP 3 787 314 A1 and US 2012/238880 A1.

### SUMMARY

The amount of ultrasonic attenuation achieved through ultrasonic absorption is proportional to the square of the ultrasonic frequency. In a conventional backing that uses the absorption attenuation method, it is impossible to sufficiently attenuate ultrasonic components within the backing that have a low frequency. As a result, ultrasound may reflect from the back face of the backing. If the reflected waves reach the piezoelectric layer, noise may appear in ultrasonic images. Further, the piezoelectric layer may be heated more in comparison with a case without the reflected waves. In order to enhance safety to the living body and protect the piezoelectric layer, it is desired to effectively dissipate heat from the piezoelectric layer.

An object of the present disclosure is to describe a backing which can provide a sufficient ultrasonic attenuation effect and heat dissipation effect, and a manufacturing method of the backing.

The ultrasonic probe according to the present invention is defined in claim 1. Further advantageous features are set out in the dependent claims.

A manufacturing method not encompassed by the wording of the claims comprises filling voids of a particle assemblage with a filler, and polishing a backing, which comprises the particle assemblage and the filler, on at least one face that is located on a living body side of the backing. The void volume ratio which indicates the ratio of the volume of the voids to the volume of the backing is within a range of 30 to 70%. The sizes of the particles are within a range of 0.05 to 2 mm.

### BRIEF DESCRIPTION OF DRAWINGS

FIG. 1 shows a cross section of an ultrasonic probe according to an embodiment, which is not an embodiment of the present invention but an example useful for its understanding, cut along X and Z axes;
FIG. 2 shows a cross section of the ultrasonic probe according to the embodiment of the present disclosure, cut along Y and Z axes;
FIG. 3 shows an enlarged cross section of a backing;
FIG. 4 shows a flowchart of a manufacturing method of the backing;
FIG. 5 shows a flowchart of a manufacturing method of the ultrasonic probe; and
FIG. 6 shows a cross section of an ultrasonic probe according to an embodiment of the present invention, cut along X and Z axes.

### DESCRIPTION OF EMBODIMENTS

Embodiments of the present disclosure are described below with reference to the attached drawings.

### (1) Overview of Embodiment

An ultrasonic probe according to an embodiment of the present disclosure comprises a piezoelectric element array, a backing, and a heat absorber. The piezoelectric element array is made from multiple piezoelectric elements and transmits ultrasound forward; that is, toward a living body. The backing is disposed behind the piezoelectric element array. The heat absorber is attached to the backing. The backing comprises a porous medium and a filler. The porous medium is made from a heat conductive material and includes voids which irregularly expand across the backing. The filler is a material which fills the voids. Ultrasound which propagates backward from the piezoelectric element array scatters and attenuates in the porous medium. Heat generated at the piezoelectric element array is conducted to the heat absorber via the porous medium.

The above configuration allows the ultrasound which propagates backward from the piezoelectric element array to scatter and attenuate in the backing. More specifically, because the voids spread irregularly; in other words, faces of the voids in the porous medium are irregularly oriented, and the ultrasound repeats irregular reflection from the faces of the voids of the porous medium. During this process, ultrasound efficiently attenuates. Because such an attenuation through scattering is completely or almost completely independent from the frequency of the ultrasound, issues of residual or reflected low frequency components inside the backing are not likely to occur. This also leads to another advantage that the thickness of the backing can be reduced.

As the porous medium is made from a heat conductive material, heat generated at the piezoelectric element array can be efficiently conducted to the heat absorber via the porous medium. As a result, because a temperature increase of the piezoelectric element array can be reduced, safety to the living body can be enhanced, and the transducer element array can be protected. The backing according to an embodiment of the present disclosure can be applied when a heat sensitive piezoelectric material is used or when it is more strongly desired to reduce a temperature increase of ultrasound transmission/reception face of the ultrasonic probe. The heat conductive material has a heat conductivity higher than that of the filler.

In an embodiment of the present disclosure, the void volume ratio, which indicates the ratio of the volume of voids to the volume of the backing, is within a range of 30 to 70%. A void volume ratio less than 30% would make it impossible to achieve sufficient ultrasound attenuation, or would require a larger backing. A void volume ratio larger than 70% would make it impossible to achieve sufficient ultrasound attenuation or heat exhaust.

In an embodiment of the present disclosure, the porous medium is a particle assemblage of multiple particles of irregular shapes (also referred to as random or inconsistent shapes). The multiple particles also have irregular sizes (also referred to as random or inconsistent sizes). With such multiple particles of irregular shapes and sizes, because the irregularity of the voids increases, the scattering ratio of ultrasound inside the backing can be increased.

In an embodiment of the present disclosure, each particle has a size within a range of 0.05 to 2 mm. When each particle has a size in this range, sufficient attenuation can be achieved in consideration of the frequency range of the applicable ultrasound. The particle sizes may be limited to within a range of 0.2 to 1 mm. The size of each irregularly-shaped particle indicates the longest dimension of the particle. In the particle assemblage, adjacent particles are in contact and united with one another. The particle assemblage is generated such that multiple heat conductive passages are formed uninterrupted from multiple piezoelectric elements to the heat absorber. In other words, the particle assemblage is a dense group of particles.

The particles comprise insulative ceramic particles. When the filler and the particles are both made from insulation materials, electric short circuits between the piezoelectric elements do not occur. Alternatively, the particle assemblage may be configured with an upper layer made from insulative particles, and a lower layer made from electrically conductive particles which have a high heat conductivity. This configuration can avoid electric short circuits and also increase the heat conductivity.

In an embodiment of the present disclosure, a hard backing element array may be disposed behind the piezoelectric element array. The back face of the hard backing element array may be attached to the front face of the porous medium. The back face of the porous medium may be attached to the front face of the heat absorber.

A backing manufacturing method related to an embodiment of the present disclosure comprises a filling process and a polishing process. In the filling process, voids of the particle assemblage including multiple particles are filled with a filler. In the polishing process, after the filler is cured, the backing that comprises the particle assemblage and the filler is polished on at least one face that is located on the living body side of the backing. The void volume ratio indicating the ratio of the volume of voids to the volume of the backing is within a range of 30 to 70%. The size of each particle is within a range of 0.05 to 2 mm.

The above configuration facilitates the manufacturing of the backing that can achieve sufficient ultrasound attenuation effects and heat conductive effects. In an embodiment of the present disclosure, the particles may have irregular sizes and shapes. The particles are made from insulation ceramic.

### (2) Detail Description of Embodiments

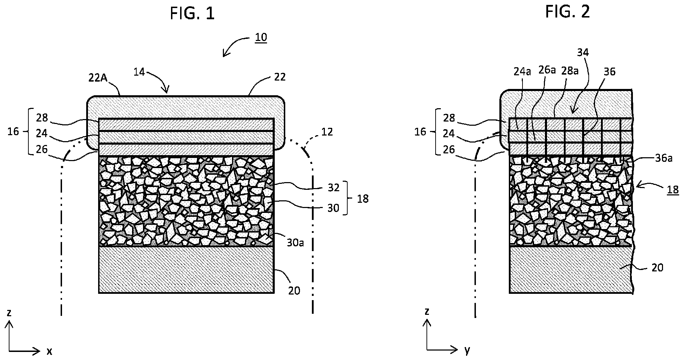

FIG. 1 shows a cross section of an ultrasonic probe 10 according to an embodiment, which is not an embodiment of the present invention but an example useful for its understanding, cut along the X and Z axes. Specifically, FIG. 1 schematically shows a head portion of the ultrasonic probe 10. The ultrasonic probe 10 is connected to the body of an ultrasonic diagnostic system (not shown). The ultrasonic probe 10 and the body of the ultrasonic diagnostic system constitute the ultrasonic diagnostic system, which is a medical apparatus used to perform an ultrasonic diagnosis of a subject. The ultrasonic probe 10 transmits ultrasound into the body of a subject and receives ultrasound reflected from within the body.

In FIG. 1, the Z axis indicates the direction to which ultrasound is transmitted. In FIG. 1, the top is directed towards the living body (front), whereas the bottom is on the opposite side from the living body (back). The Y axis (not shown in FIG. 1) shows a scanning direction. The X axis shows an elevation direction; i.e., the direction perpendicular to the Z and Y axes.

The ultrasonic probe 10 comprises a housing 12 and a transducer assembly 14, which is almost entirely enclosed in the housing 12. The housing 12 may be, for example, a hollow plastic case. The transducer assembly 14 comprises a stack of layers 16, a backing 18, a heat exhaust block 20, and an acoustic lens 22.

In the embodiment shown in FIG. 1, the stack of layers 16 comprises a piezoelectric layer 24, a hard backing layer 26 disposed behind the piezoelectric layer 24, and a matching layer 28 disposed in front of the piezoelectric layer 24. The piezoelectric layer 24 comprises multiple piezoelectric elements arranged along the Y axis (piezoelectric element array). The hard backing layer 26 comprises multiple hard backing elements arranged along the Y axis (hard backing element array). The matching layer 28 comprises multiple matching elements arranged along the Y axis (matching element array). Two or more matching layers may be disposed in front of the piezoelectric layer 24. For example, the matching element array may be provided as the first matching layer, and a second matching layer may be disposed on top of the first matching layer.

The acoustic lens 22 is disposed on the living body side of the stack of layers 16. The front face of the acoustic lens 22 is an ultrasound transmission/reception face 22A which is to be held in contact with the surface of a living body. With the contact being maintained, ultrasound is transmitted and received. An operator (medical practitioner, medical technologist, or the like) holds the ultrasonic probe 10 by the housing 12. The ultrasonic probe 10 is a mobile ultrasound transceiver.

The backing 18 is disposed behind the stack of layers 16. The backing 18 functions to scatter and attenuate undesired backward ultrasound, and to conduct the heat generated at the piezoelectric layer 24 to the heat exhaust block 20. More specifically, the backing 18 comprises a particle assemblage 30 which is a porous medium, and a filler 32 which fills voids of the particle assemblage 30. The backing 18 is described in detail below.

The heat exhaust block 20 serving as a heat absorber is disposed behind the backing 18. The heat exhaust block 20 is a heat conductor that absorbs or releases heat. The heat exhaust block 20 may have any of a variety of shapes.

The front face of the backing 18 is attached to the back face of the hard backing layer 26. The back face of the backing 18 is attached to the heat exhaust block 20. When the hard backing layer 26 is not provided, the back face of the piezoelectric layer 24 is attached to the front face of the backing 18.

The piezoelectric layer 24 and the hard backing layer 26 may be united with each other to provide a resonator. The thickness of the piezoelectric layer 24 and the thickness of the hard backing layer 26 are adjusted such that the thickness of the resonator is λ/2, where λ is a wavelength corresponding to the center frequency of ultrasound.

A portion of ultrasound that propagates backward from the back face of the piezoelectric layer 24 is reflected from the back face of the hard backing layer 26 to the front (toward the living body). However, the backward ultrasound partially leaks through the hard backing layer 26 and propagates further into the backing 18. In order to avoid the leaked ultrasound from causing noise, the backing 18 attenuates the leaked ultrasound.

The piezoelectric layer 24 is made from a piezoelectric material, such as single-crystal PMN-PT or single-crystal PZN-PT. Because these materials are relatively sensitive to heat, exhaust of heat from the piezoelectric layer 24 is important. The exhaust of heat from the piezoelectric layer 24 is also important in order to reduce the temperature increase of the ultrasound transmission/reception face 22A. The piezoelectric layer 24 may be made from polycrystal PZT. The acoustic impedance of the piezoelectric layer 24 may be, for example, 25 to 30 MRayls. The hard backing layer 26 may be made from, for example, a metal whose acoustic impedance may be, for example, 80 to 120 MRayls. The average acoustic impedance of the backing 18 may be, for example, 3 to 4 MRayls.

The backing 18 may have the thickness (dimension along the Z axis) within a range of, for example, 5 to 20 mm. The particle assemblage 30 of the backing 18 comprises a large number of particles 30a densely clustered together. The particle assemblage 30 includes irregular voids which expand across the backing 18. The respective spaces in the voids have random shapes. The void volume ratio which indicates the ratio of the volume of voids to the volume of the backing 18 (i.e., the particle assemblage 30) is within a range of, for example, 30 to 70%.

The voids are filled with the filler 32. In other words, the backing 18 is made by impregnating the particle assemblage 30 with the filler 32. The filler 32 contains a resin, and a filler material added to the resin. As the filler material, a viscosity adjusting filler, a heat conductivity adjusting filler, an acoustic impedance adjusting filler, and the like are known. The average diameter of the particles of the filler material is within a range of, for example, 1 to 15 µm. The particle assemblage 30 has a heat conductivity higher than the average heat conductivity of the filler 32 and functions as a heat conductor.

More specifically, respective particles 30a of the particle assemblage 30 are solid particles of an insulative ceramic material, having irregular shapes and sizes. The shapes and sizes of the particles are thus random. The size of each particle may be within a range of, for example, 0.05 to 2 mm, more preferably 0.2 to 1 mm. The size of each particle indicates the longest dimension of the particle. A large number of irregularly-shaped particles may be obtained by crushing a ceramic material. Particles of a size within a certain range can be extracted by classifying the particles after the crushing; specifically, by sieving the particles.

If particles are too small, reflection of ultrasound from surfaces of particles becomes too weak, whereas, if too large, the reflection becomes too intense. The particle assemblage 30 is prepared such that ultrasound in the backing 18 sufficiently scatters to exhibit efficient attenuation of ultrasound in the backing 18.

The backing according to the present disclosure attenuates ultrasound using a reflection attenuation method which can attenuate ultrasound almost independently from the frequencies of ultrasound. Embodiments of the present disclosure can thus solve issues of residual low frequency components.

The particles 30a may be made from, for example, alumina (Al₂O₃), zirconia (ZrO₂), barium titanate (BaTiO₃), silicon carbide (SiC), aluminum nitride (AlN), or boron nitride (BN). The resin may be, for example, an epoxy resin, a urethane resin, an acrylic resin, a phenolic resin, or a silicone resin. A filler material to adjust viscosity, attenuation, and heat conductive ratio is added to the resin. The filler material may be, for example, a silicon dioxide filler, a tungsten filler, a tungsten silicide filler, a silicon carbide filler, a boron nitride filler, or a silicone rubber filler. The heat exhaust block may be made from, for example, a metal such as copper, aluminum, or iron, or carbon, graphite, ceramic, or the like. As the heat exhaust block, a heatsink may be provided.

As a large number of heat conductive particles 30a exist randomly and densely between the hard backing layer 26 and the heat exhaust block 20 as shown in FIG. 1, heat generated at the piezoelectric layer 24 can be efficiently conducted to the heat exhaust block 20. The reflection of ultrasound at the back face of the backing 18 does not occur or becomes weak enough to be ignored. Because this eliminates the need to match the acoustic impedance of the heat exhaust block 20 to that of the backing 18, the heat exhaust block 20 can be made from any of a variety of materials.

FIG. 2 is a cross section of the ultrasonic probe according to an embodiment of the present disclosure, cut along the Y and Z axes. As described above, the stack of layers 16 includes the piezoelectric layer 24, the hard backing layer 26, and the matching layer 28. The piezoelectric layer 24 is a piezoelectric element array which includes multiple piezoelectric elements 24a arranged along the Y axis. The hard backing layer 26 is a hard backing element array which includes multiple hard backing elements 26a arranged along the Y axis. The matching layer 28 is a matching element array which includes multiple matching elements 28a arranged along the Y axis.

In other words, the stack of layers 16 includes element stacks 34 which are arranged along the Y axis. The respective element stacks 34 include the piezoelectric elements 24a, the hard backing elements 26a, and the matching elements 28a. During the manufacturing process of the ultrasonic probe, a stack of layers which is not yet divided is formed on the front face of the backing 18. The stack of layers is then diced, during which multiple separation gaps 36 are formed and the stack of layers is divided into the individual element stacks 34. One end 36a of each separation gap 36 extends into the backing 18. The separation gaps 36 are filled with a filler material.

The hard backing elements 26a are thermally connected to the heat exhaust block 20 via the particle assemblage 30 in the backing 18. The particle assemblage 30 is configured such that multiple heat conductive passages are formed uninterruptedly between multiple hard backing elements 26a and the heat exhaust block 20.

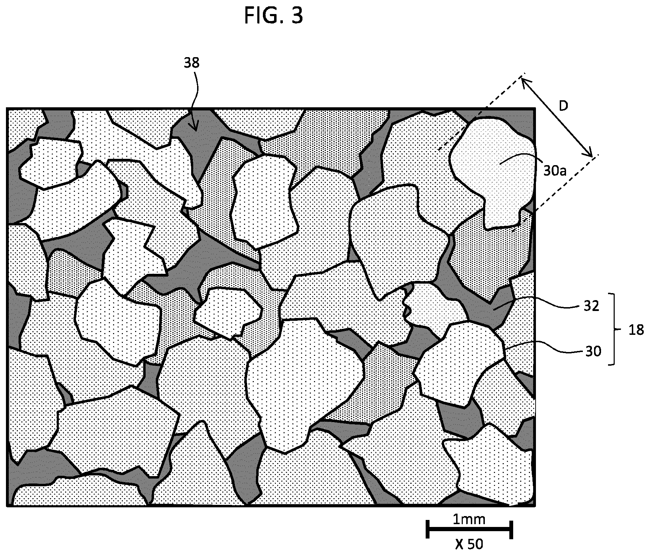

FIG. 3 shows an enlarged cross section of the backing 18. FIG. 3 simulates a microscope image. The magnification required to obtain such a microscope image is 50x. Relatively bright areas represent the particles 30a of the particle assemblage 30. Relatively dark areas represent voids 38 which are filled with the filler 32. Although it may not be clear from FIG. 3, adjacent particles are connected with one another in the particle assemblage 30. The connected particles are united together.

The particles 30a of the particle assemblage 30 have irregular shapes and sizes. In other words, the shapes and sizes are random. Ultrasound is diffused on the surfaces of the particles 30a within the backing 18. Ultrasound efficiently attenuates while being repeatedly diffused. "D" indicates the longest dimension of the respective three-dimensional particle 30a.

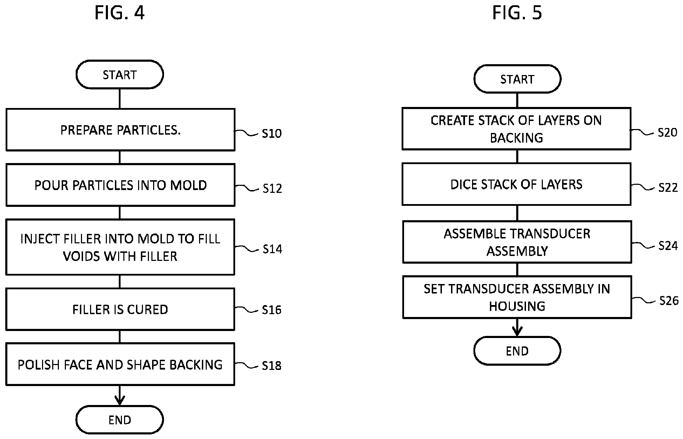

FIG. 4 shows a flowchart of a backing manufacturing method according to an embodiment of the present disclosure. In S10, particles are prepared by crushing ceramic. The particles are then classified by size. In S12, the particles are poured into a mold to solidify the particles into a particle assemblage. During this process, pressure or heat may be applied to the particles.

In S14, a filler is injected into the mold such that the particle assemblage is impregnated with the filler. Alternatively, this process may be performed outside of the mold. The particle assemblage includes voids which are filled with the filler. During this filling process, the filler is injected to sufficiently remove air bubbles from the voids. S16 is a resin curing process. In this process, heat and/or pressure may be applied. The preparation of the backing completes when the resin is cured.

In S18, respective faces of the backing are polished. The backing may also be shaped as required in S18. In the polishing process, at least the face that is to be attached to the stack of layers (more specifically, the hard backing layer) is polished.

FIG. 5 shows an example of a manufacturing method of an ultrasonic probe. In S20, a stack of layers which is not yet divided is formed. In S22, the stack of layers is diced into individual element stacks. In this dicing process, scribe lines are formed and filled with a filler material. In S24, a transducer assembly which includes the backing, the stack of layers, and the acoustic lens is assembled. In S26, the transducer assembly is set in a housing.

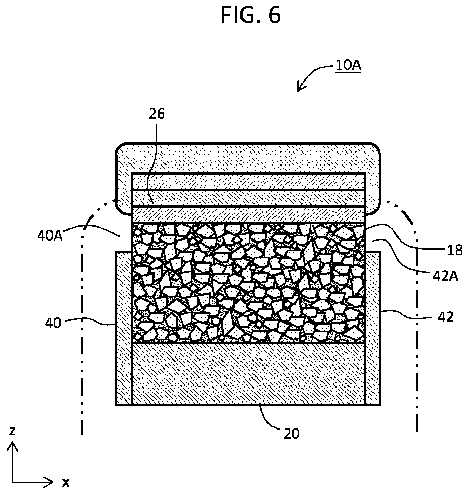

FIG. 6 shows an ultrasonic probe 10A according an embodiment of the present invention. A side plate 40 and another side plate 42 are respectively attached to a first side face and a second side face of the backing 18. The side plates 40, 42 are heat conductive plates which may be made from, for example, a metal. The heat which has been absorbed by the side plates 40, 42 is conducted to the heat exhaust block 20.

Gaps (exposed portions) 40A, 42A are respectively provided at the first side face and the second side face of the backing 18. If the first and the second faces were entirely covered by the side plates 40, 42, reflected waves from the surfaces of the side plates 40, 42 could be problematic. In order to avoid such a problem, the gaps 40A, 42A are provided. The first and second side faces are oriented perpendicular to the X axis. Another two side plates may be provided for the third and fourth side faces which are oriented perpendicular to the Y axis.

Although in the ultrasonic probes in the above embodiments the piezoelectric elements are arranged in a flat plane, the piezoelectric elements may be arranged in a curved plane. The configurations according to the present disclosure may be applied to an ultrasonic probe which includes a two-dimensional vibration element array.

## Claims

1. An ultrasonic probe comprising:
a piezoelectric element array (24) including a plurality of piezoelectric elements and configured to transmit ultrasound forward, toward a living body; and
a backing (18) disposed behind the piezoelectric element array (24);
wherein the backing (18) comprises:
a porous medium (30) made from a heat conductive material; and
a filler (32) filling voids (38) irregularly expanding in the porous medium across the backing (18), and
wherein ultrasound propagating backward from the piezoelectric element array (24) scatters and attenuates in the porous medium (30),
wherein the ultrasonic probe further comprises:
a heat absorber (20) attached to the backing (18); and
first and second side plates (40, 42) attached to respective first and second side faces of the backing (18); and
wherein the first and second side faces are not entirely covered by the first and second side plates (40, 42) but are provided with respective first and second exposed portions (40A, 42A) for reducing the reflection of ultrasound occurring at the surfaces of the first and second side plates (40, 42);
**characterized in that** the first and second side plates (40, 42) are in contact with respective first and second sides of the heat absorber (20) such that heat generated at the piezoelectric element array (24) is conducted to the heat absorber (20) via the porous medium (30) and via the first and second side plates (40, 42); and
the porous medium (30) is a particle assemblage comprising insulative ceramic particles (30a) connected and united together.

2. The ultrasonic probe according to claim 1, wherein
the void volume ratio which indicates the ratio of the volume of voids (38) to the volume of the backing (18) is within a range of 30 to 70%.

3. The ultrasonic probe according to claim 1, wherein
the insulative ceramic particles (30a) have irregular shapes.

4. The ultrasonic probe according to claim 1 or 3, wherein
the insulative ceramic particles (30a) have irregular sizes.

5. The ultrasonic probe according to claim 1, 3 or 4, wherein
the sizes of the insulative ceramic particles (30a) are within a range of 0.05 to 2 mm.

6. The ultrasonic probe according to any preceding claim, wherein
a hard backing element array (26) is disposed behind the piezoelectric element array (24),
a back face of the hard backing element array (26) and a front face of the porous medium (30) are attached to each other, and
a back face of the porous medium (30) and a front face of the heat absorber (20) are attached to each other.

## Patentansprüche

1. Ultraschallsonde, umfassend:
eine piezoelektrische Elementanordnung (24), die mehrere piezoelektrische Elemente enthält und so konfiguriert ist, dass sie Ultraschall vorwärts, zu einem lebenden Körper hin, überträgt; und
eine Rückenschicht (18), die hinter der piezoelektrischen Elementanordnung (24) angeordnet ist;
wobei die Rückenschicht (18) umfasst:
ein poröses Medium (30), das aus einem wärmeleitenden Material hergestellt ist; und
einen Füllstoff (32), der Hohlräume (38), die in dem porösen Medium über die Rückenschicht (18) unregelmäßig expandieren, füllt, und
wobei Ultraschall, der von der piezoelektrischen Elementanordnung (24) rückwärts propagiert, in dem porösen Medium (30) streut und sich dämpft,
wobei die Ultraschallsonde ferner umfasst:
einen Wärmeabsorber (20), der an der Rückenschicht (18) angebracht ist; und
erste und zweite Seitenplatten (40, 42), die an jeweiligen ersten und zweiten Seitenflächen der Rückenschicht (18) angebracht sind; und
wobei die ersten und zweiten Seitenflächen nicht vollständig durch die erste und zweite Seitenplatten (40, 42) abgedeckt sind, sondern mit jeweiligen ersten und zweiten freiliegenden Abschnitten (40A, 42A) zum Reduzieren der Reflexion von Ultraschall, die an den Oberflächen der ersten und zweiten Seitenplatten (40, 42) auftritt, versehen sind;
**dadurch gekennzeichnet, dass**
die ersten und zweiten Seitenplatten (40, 42) mit jeweiligen ersten und zweiten Seiten des Wärmeabsorbers (20) so in Kontakt stehen, dass Wärme, die an der piezoelektrischen Elementanordnung (24) erzeugt wird, via das poröse Medium (30) und via die ersten und zweiten Seitenplatten (40, 42) zu dem Wärmeabsorber (20) geleitet wird; und
das poröse Medium (30) eine Partikelansammlung ist, die Isolierkeramikpartikel (30a), die miteinander verbunden und vereinigt sind, umfasst.

2. Ultraschallsonde nach Anspruch 1, wobei
das Hohlraumvolumenverhältnis, das das Verhältnis des Volumens von Hohlräumen (38) zu dem Volumen der Rückenschicht (18) angibt, innerhalb eines Bereichs von 30 bis 70 % liegt.

3. Ultraschallsonde nach Anspruch 1, wobei
die Isolierkeramikpartikel (30a) unregelmäßige Formen aufweisen.

4. Ultraschallsonde nach Anspruch 1 oder 3, wobei
die Isolierkeramikpartikel (30a) unregelmäßige Größen aufweisen.

5. Ultraschallsonde nach Anspruch 1, 3 oder 4, wobei
die Größen der Isolierkeramikpartikel (30a) innerhalb eines Bereichs von 0,05 bis 2 mm liegen.

6. Ultraschallsonde nach einem der vorhergehenden Ansprüche, wobei
eine harte Rückenschicht-Elementanordnung (26) hinter der piezoelektrischen Elementanordnung (24) angeordnet ist,
eine Rückfläche der harten Rückenschicht-Elementanordnung (26) und eine Vorderfläche des porösen Mediums (30) aneinander angebracht sind, und
eine Rückfläche des porösen Mediums (30) und eine Vorderfläche des Wärmeabsorbers (20) aneinander angebracht sind.

## Revendications

1. Sonde ultrasonore comprenant :
un réseau d'éléments piézoélectriques (24) incluant une pluralité d'éléments piézoélectriques et configuré pour transmettre des ultrasons vers l'avant, vers un corps vivant ; et
un support (18) disposé derrière le réseau d'éléments piézoélectriques (24) ;
dans laquelle le support (18) comprend :
un milieu poreux (30) constitué d'un matériau thermoconducteur ; et
un charge (32) remplissant des vides (38) s'étendant de manière irrégulière dans le milieu poreux à travers le support (18), et
dans laquelle les ultrasons se propageant vers l'arrière à partir du réseau d'éléments piézoélectriques (24) se diffusent et s'atténuent dans le milieu poreux (30),
dans laquelle la sonde ultrasonore comprend en outre :
un absorbeur de chaleur (20) fixé au support (18) ; et
des première et deuxième plaques latérales (40, 42) fixées à des première et deuxième faces latérales respectives du support (18) ; et
dans laquelle les première et deuxième faces latérales ne sont pas entièrement couvertes par les première et deuxième plaques latérales (40, 42) mais sont pourvues de première et deuxième parties exposées respectives (40A, 42A) pour réduire la réflexion des ultrasons se produisant aux surfaces des première et deuxième plaques latérales (40, 42) ;
**caractérisée en ce que**
les première et deuxième plaques latérales (40, 42) sont en contact avec des première et deuxième côtés respectifs de l'absorbeur de chaleur (20) de sorte que la chaleur générée au niveau du réseau d'éléments piézoélectriques (24) est conduite vers l'absorbeur de chaleur (20) via le milieu poreux (30) et via les première et deuxième plaques latérales (40, 42) ; et
le milieu poreux (30) est un assemblage de particules comprenant des particules céramiques isolantes (30a) reliées et unies entre elles.

2. Sonde ultrasonore selon la revendication 1, dans laquelle
le rapport de volume de vides qui indique le rapport du volume de vides (38) au volume du support (18) est dans une plage de 30 à 70 %.

3. Sonde ultrasonore selon la revendication 1, dans laquelle
les particules céramiques isolantes (30a) ont des formes irrégulières.

4. Sonde ultrasonore selon la revendication 1 ou la revendication 3, dans laquelle
les particules céramiques isolantes (30a) ont des tailles irrégulières.

5. Sonde ultrasonore selon la revendication 1, la revendication 3 ou la revendication 4, dans laquelle
les tailles des particules céramiques isolantes (30a) sont dans une plage de 0,05 à 2 mm.

6. Sonde ultrasonore selon l'une quelconque des revendications précédentes, dans laquelle
un réseau d'éléments de support rigide (26) est disposé derrière le réseau d'éléments piézoélectriques (24),
une face arrière du réseau d'éléments de support rigide (26) et une face avant du milieu poreux (30) sont fixées l'une à l'autre, et
une face arrière du milieu poreux (30) et une face avant de l'absorbeur de chaleur (20) sont fixées l'une à l'autre.
